# FASCICULE DE BREVET EUROPEEN

(11) **EP 0 575 231 B1**
(45) Date de publication et mention de la délivrance du brevet: **06.03.1996**
(21) Numéro de dépôt: 93401511.6
(22) Date de dépôt: 11.06.1993
(51) Int. Cl.: A61B 17/15, A61B 17/56

(54) **Dispositif de stabilisation d'un moyen de coupe pour la pose d'une prothèse en chirurgie osseuse**
Schneidemittelstabilisierungsvorrichtung zum Einsetzen einer Prothese in der Knochenchirurgie
Cutting means stabilizing apparatus for implanting a prosthesis in bone surgery

(30) Priorité: 16.06.1992 FR 9207292
(43) Date de publication de la demande: 22.12.1993
(73) Titulaire: DEVELOPPEMENT IMPLANTS ORTHOPEDIQUES ET MEDICAUX, F-25460 Etupes (FR); PROTEK SYNTHES, F-25460 Etupes (FR)
(72) Inventeur: Aebi, Jürg, F-75116 Paris (FR); Beaufils, Philippe, F-75116 Paris (FR); De Lestang, Michel, F-75116 Paris (FR); Gaffuri, Jean-Gilles, F-75116 Paris (FR); Hourlier, Hervé, F-75116 Paris (FR); Lallement, Jean-Jacques, F-75116 Paris (FR); Legroux, Philippe, F-75116 Paris (FR); Levai, Jean-Paul, F-75116 Paris (FR); Pondaven, Gérard, F-75116 Paris (FR); Schuster, Pierre, F-75116 Paris (FR); Vergnat, Christian, F-75116 Paris (FR); Bouraly, Jean-Pierre, F-75116 Paris (FR)
(74) Mandataire: Lemoyne, Didier

(56) Documents cités:
- EP-A- 243 109
- EP-A- 0 384 562
- WO-A-91/19461
- DE-A- 2 224 214
- DE-U- 9 017 101
- US-A- 4 487 203
- US-A- 4 927 422
- US-A- 5 053 037
- US-A- 5 129 909

## Description

La présente invention concerne un dispositif de stabilisation axiale d'un moyen de coupe utilisé pour effectuer au moins une coupe à une extrêmité d'un os en vue de la pose d'une prothèse en chirurgie osseuse. Elle concerne également un outillage destiné à être utilisé en chirurgie osseuse.

L'invention trouve une application particulièrement avantageuse, mais non exclusive, dans le domaine de la pose de prothèses du genou

D'une manière générale, la pose d'une prothèse en chirurgie osseuse exige la mise en oeuvre d'un outillage, dit matériel ancillaire, permettant de préparer la pose de la prothèse proprement dite. Dans le cas d'une prothèse fémorale du genou présentant un certain nombre de surfaces de contact internes planes, il faut effectuer à l'extrémité distale du fémur des coupes correspondantes de façon à garantir une mise en place parfaite de la prothèse sur l'os ainsi aménagé. Dans ce but, on utilise un moyen de coupe, gabarit ou boitier multicoupe, qui doit être maintenu en position stable à l'extrémité distale du fémur. La technique habituellement employée consiste à percer l'échancrure intercondyliaire fémorale pour introduire partiellement dans le canal intra-médullaire une tige de centrage, dite centro-médullaire, destinée à reçevoir ledit moyen de coupe. Ce procédé assure effectivement un centrage satisfaisant du moyen de coupe. Toutefois, compte tenu du fait que ladite tige de centrage est implantée directement dans l'os, la stabilité axiale de la tige,et donc du moyen de coupe, n'est pas suffisante.En effet, la structure de l'os en cet endroit est très variable: soit relativement compacte, soit désorganisée et donc sans résistance mécanique.

Pour remédier à cette difficulté, on a recours à diverses méthodes connues.Il s'agit par exemple du brochage du moyen de coupe sur la partie distale du fémur qui permet de maintenir en position ledit moyen de coupe sans pourtant réaliser un véritable appui contre l'os, ce qui a pour inconvénient de rendre possible le déplacement du moyen de coupe sous l'effet des vibrations produites par les scies oscillantes utilisées lors des opérations de coupe.

On peut également munir la tige de centrage de moyens d'ancrage du type harpon constitués par des ailettes dentées.Outre le fait qu'il est peu fiable au niveau de l'ancrage, un tel système a le désavantage d'être destructif puisque l'extraction de la tige se fait par arrachage.

On connaît des documents EP-A-243 109 et US-A-4 487 203 des dispositifs pour effectuer des coupes à l'extrémité d'un os, comportant dans chaque cas une tige de centrage intra-médullaire.

Le brevet américain prévoit cependant un moyen de stabilisation du moyen de coupe par rapport à la tige de centrage qui est, dans ce cas là, non rectiligne. L'extrémité de la partie proximale coudée de cette tige est en effet fixée au boîtier de coupe par une vis dont la fonction est simplement de solidariser la tige au boîtier.

Aussi, le problème technique à résoudre par l'objet de la présente invention est de proposer un dispositif de stabilisation axiale d'un moyen de coupe utilisé pour effectuer au moins une coupe à une extrémité d'un os en vue de la pose d'une prothèse en chirugie osseuse , ledit dispositif comportant un moyen de centrage, constitué par une tige de centrage, introduite partiellement dans ledit os et destinée à reçevoir ledit moyen de coupe, un moyen d'application et de mise en compression dudit moyen de coupe contre l'extrémité de l'os étant monté à l'extrémité proximale de la tige de centrage, dispositif qui permettrait d'obtenir une stabilité parfaite du moyen de coupe dans le sens axial tout en assurant une extraction de la tige sans dommage pour les tissus osseux .

La solution au problème technique posé consiste, selon la présente invention , en ce que ladite tige de centrage est munie d'un filetage d'ancrage de manière à réaliser un tire-fond stabilisé axialement dans une direction parallèle à l'axe de la tige.

Ainsi,le filetage d'ancrage dont est munie la tige garantit une stabilité axiale parfaite du moyen de coupe et l'extraction de la tige formant tire-fond est réalisée de façon très simple par dévissage de la tige sans que cette opération n'entraîne de dégâts osseux.

Bien entendu,le dispositif conforme à l'invention peut-être mis en oeuvre avec tous types de moyen de coupe, que ce soit des gabarits à coupe unique placés successivement sur le même tire-fond, ou encore un seul boitier permettant d'effectuer toutes les coupes requises.

Ce dernier moyen de coupe est particulièrement avantageux puisqu'il assure une corrélation idéale des coupes successives. Pour obtenir un résultat parfait, il suffit de maintenir le moyen de coupe dans une position axiale déterminée. Pour cela, il est prévu, selon l'invention,que ledit dispositif comporte également un moyen d'application et de mise en compression axiales dudit moyen de coupe contre l'extrémité de l'os, c'est-à-dire un moyen dont l'action est dirigée parallèlement à l'axe de la tige pénétrant dans le canal intra-médullaire, de manière à coopérer avec le filetage d'ancrage de la tige de centrage afin d'assurer une stabilisation maximale du moyen de coupe. Celui-ci étant ainsi maintenu en permanence en appui contre l'os, il constitue une référence géométrique fixe pour réaliser les coupes successives.

La description qui va suivre en regard du dessin annexé, donné à titre d'exemple non limitatif, fera bien comprendre en quoi consiste l'invention et comment elle peut être réalisée.

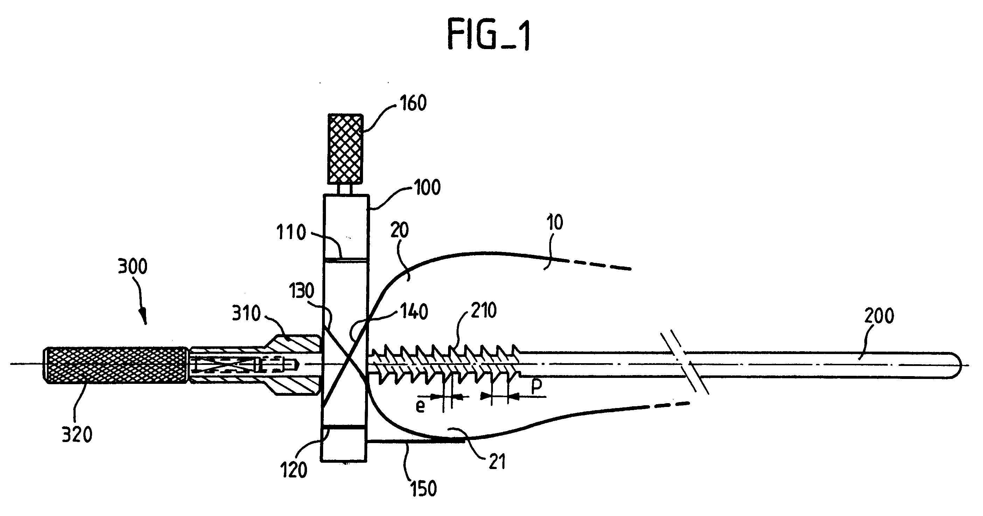

La figure 1 est une vue en coupe d'un outillage de pose de prothèse fémorale du genou comportant un dispositif de stabilisation longitudinale conforme à l'invention.

La figure 1 montre, en coupe, un outillage ,appelé aussi matériel ancillaire, pour la pose d'une prothèse fémorale du genou. Ce matériel ancillaire comporte un moyen 100 de coupe qui dans l'exemple de la figure 1 est un boitier permettant de réaliser une pluralité de coupes à l'extrémité 20 d'un os 10, ici le fémur, à l'aide de fentes 110, 120, 130, 140 à travers lesquelles est placée successivement une scie oscillante non représentée. On notera que le boitier 100 de coupe fémoral comporte des pattes postérieures 150 qui sont mises en appui sur les condyles postérieurs 21 du fémur 10 en actionnant une vis molettée 160 de réglage antéro-postérieur.

Comme le montre la figure 1,l'ancillaire de pose comprend un dispositif de stabilisation axiale du boîtier 100, comportant une tige intramédullaire 200 de centrage introduite partiellement dans le canal centro-médullaire du fémur 10 et destinée à reçevoir le boitier 100 de coupe fémorale.

Conformément à la figure 1, la tige 200 est munie d'un filetage 210 d'ancrage réalisant ainsi un tire-fond stabilisé axialement dans une direction parallèle à l'axe de la tige 200.

Le boitier 100 se trouve donc parfaitement centré par rapport à l'axe du fémur 10 et, pour le stabiliser dans le sens axial, il suffit de le solidariser avec la tige 200 formant tire-fond. A cet effet, le dispositif illustré à la figure 1 comporte également un moyen 300 d'application et de mise en compression du boitier 100 de coupe fémorale contre l'extrémité 20 de l'os 10.

Ainsi qu'on peut le voir sur la figure 1, ledit moyen 300 d'application et de mise en compression axiales est constitué par une vis moletée 320 montée à l'extrémité proximale du tire-fond 200 et entraînant une entretoise 310 destinée à venir en appui contre le boîtier 100 de coupe.

On peut observer sur la figure 1 que ledit filetage 210 d'ancrage est aménagé dans la partie proximale de la tige 200 formant tire-fond, la longueur distale de la tige servant de moyen de guidage à travers le canal centro-médullaire.

De façon pratique, la longueur du filetage est compris entre 10 et 50% du tire-fond 200. Du fait de sa longueur limitée , il est préférable de prévoir une forme de filetage susceptible de fournir un volume d'ancrage apte à immobiliser axialement la tige 200 dans l'os 10. Dans ce but, il est proposé un filetage à épaisseur e faible devant le pas p, le rapport e/p devant être au plus égal à 25%. On réalise ainsi un filet présentant un tranchant très fin avec un espace susceptible de loger un stock osseux suffisant compte tenu du fait que le milieu dans lequel le tire-fond 200 est introduit, est relativement peu résistant.

Les demandeurs ont établi qu'un bon compromis entre un volume d'ancrage suffisant et un filetage de diamètre compatible avec les caractéristiques dimensionnelles de l'os était obtenu lorsque le rapport du diamètre du filetage 210 au diamètre de la tige 200 est compris entre 1,5 et 1,8.

Le filetage est réalisé par exemple par usinage d'une tige en acier austénitique à durcissement thermique.

## Revendications

1. Dispositif de stabilisation axiale d'un moyen (100) de coupe utilisé pour effectuer au moins une coupe à une extrémité (20) d'un os (10) en vue de la pose d'une prothèse en chirurgie osseuse, ledit dispositif comportant un moyen (200) de centrage introduit partiellement dans ledit os (10) et destiné à recevoir ledit moyen (100) de coupe, un moyen (300) d'application et de mise en compression dudit moyen (100) de coupe contre ladite extrémité (20) de l'os (10) étant monté à l'extrémité proximale du moyen de centrage (200), caractérisé en ce que ledit moyen (200) est constitué d'une tige (200) de centrage munie d'un filetage (210) d'ancrage de manière à réaliser un tire-fond stabilisé axialement dans une direction parallèle à l'axe de la tige (200), et en ce que l'application et la mise en compression dudit moyen (100) de coupe contre l'extrémité (20) de l'os (10) est effectuée axialement le long de la tige (200) par ledit moyen (300) d'application et de mise en compression.

2. Dispositif selon la revendication 1, caractérisé en ce que ledit moyen d'application et de mise en compression axiale est constitué par une vis moletée (320) montée à l'extrémité proximale de ladite tige (200) et entraînant une entretoise (310) destinée à venir en appui contre le moyen (100) de coupe.

3. Dispositif selon l'une des revendications 1 ou 2, caractérisé en ce que ledit filetage (210) d'ancrage est aménagé dans la partie proximale de la tige (200) formant tire-fond.

4. Dispositif selon l'une quelconque des revendications 1 à 3, caractérisé en ce que ledit filetage (210) présente, sur une distance inférieure à 50% de la tige (200) formant tire-fond, un volume d'ancrage apte à immobiliser axialement ladite tige dans l'os (10).

5. Dispositif selon la revendication 4, caractérisé en ce que le rapport de l'épaisseur au pas dudit filetage (210) est au plus égal à 25%.

6. Outillage destiné à être utilisé en chirurgie osseuse, caractérisé en ce qu'il comporte un dispositif selon l'une quelconque des revendications 1 à 5.

## Claims

1. Device for axially stabilising a cutting means (100) used to make at least one cut at one end (20) of a bone (10) in readiness for the fitting of a prosthesis during bone surgery, the said device having a centring means (200) partially inserted into the bone (10) and designed to receive the cutting means (100), a means (300) for applying the said cutting means (100) and compressing it against the end (20) of the bone (10), being mounted on the end next to the said centring means (200), characterised in that the said means (200) is made up of a centring rod (200) provided with a fixing thread (210) so as to form a foundation bolt axially stabilised in a direction parallel to the axis of the rod (200), and in that the said cutting means (100) is applied to and pushed axially along the rod (200) against the end (20) of the bone (10) by means of the said application and compressing means (300).

2. Device as claimed in claim 1, characterised in that the said application and axial compressing means is made up of a milled screw (320) mounted on the end next to the said rod (200) to operate a cross-piece (310), causing it to move to bear against the cutting means (100).

3. Device as claimed in one of claims 1 or 2, characterised in that the said fixing thread (210) is arranged in the portion next to the rod (200) forming the foundation bolt.

4. Device as claimed in any one of claims 1 to 3, characterised in that the said fixing thread (210) has, over a distance of less than 50% of the rod (200) forming the foundation bolt, a fixing volume designed to immobilise the said rod axially in the bone (10).

5. Device as claimed in claim 4, characterised in that the ratio of the thickness to the pitch of the said thread (210) is equal to at least 25%.

6. Equipment designed for use during bone surgery, characterised in that it comprises a device as claimed in any one of claims 1 to 5.

## Patentansprüche

1. Vorrichtung zur axialen Stabilisierung einer Schneidanordnung (100) zur Durchführung wenigstens eines Schnittes an einem Ende (20) eines Knochens (10) zum Einsetzen einer Prothese in der Knochenchirurgie, wobei die Vorrichtung eine Zentrieranordnung (200) aufweist, die teilweise in den Knochen (20) eingesetzt ist und zur Aufnahme der Schneidanordnung dient und eine Anordnung (300) zum Einsetzen und zur Druckbeaufschlagung der Schneidanordnung (100) an das Ende (20) des Knochens (10) aufweist, die an dem der Zentrieranordnung (200) zugewandten Ende vorgesehen ist, dadurch gekennzeichnet, daß die Anordnung (200) aus einem Zentrierstab besteht, der dergestalt mit einem Verankerungsgewinde versehen ist, daß er einen Verankerungsbolzen bildet, der in einer Richtung parallel zur Achse des Stabes (200) axial stabilisiert ist und daß das Einsetzen und die Druckbeaufschlagung der Schneidanordnung (100) bezüglich des Endes (20) des Knochens (10) axial entlang des Stabes (200) mittels der Anordnung (300) zum Einsetzen und zur Druckbeaufschlagung erfolgt.

2. Vorrichtung nach Anspruch 1, dadurch gekennzeichnet, daß die Anordnung zum Einsetzen und zur axialen Druckbeaufschlagung aus einer geriffelten Schraube (320) besteht, die an dem dem Stab (200) zugewandten Ende angeordnet ist und eine Verstrebung (310) antreibt, welche sich auf der Schneidanordnung (100) abstützt.

3. Vorrichtung nach einem der Ansprüche 1 oder 2, dadurch gekennzeichnet, daß das Verankerungsgewinde (210) in dem dem Stab (200) zugewandten Abschnitt ausgebildet ist, der den Verankerungsbolzen bildet.

4. Vorrichtung nach einem der Ansprüche 1 bis 3, dadurch gekennzeichnet, daß das Gewinde (210) über eine Länge, die kleiner ist als 50% des den Verankerungsbolzen bildenden Stabes (200), ein Verankerungsvolumen darstellt, das in der Lage ist, den Stab im Knochen (10) axial festzulegen.

5. Vorrichtung nach Anspruch 4, dadurch gekennzeichnet, daß das Verhältis von Dicke zu Steigung des Gewindes (210) höchstens gleich 25% ist.

6. Werkzeug zur Verwendung in der Knochenchirurgie, dadurch gekennzeichnet, daß es eine Vorrichtung nach einem der Ansprüche 1 bis 5 aufweist.
